# EUROPÄISCHE PATENTSCHRIFT

(11) **EP 3 630 229 B1**
(45) Veröffentlichungstag und Bekanntmachung des Hinweises auf die Patenterteilung: **03.04.2024**
(21) Anmeldenummer: 18728331.2
(22) Anmeldetag: 22.05.2018
(51) Int. Cl.: A61M 5/168

(54) **VOLUMETRISCHE PUMPE MIT SENSORSYSTEM**
VOLUMETRIC PUMP WITH SENSOR SYSTEM
POMPE VOLUMÉTRIQUE AVEC SYSTÈME DE CAPTEUR

(30) Priorität: 23.05.2017 DE 102017111301
(43) Veröffentlichungstag der Anmeldung: 08.04.2020
(73) Patentinhaber: B. Braun Melsungen AG, 34212 Melsungen (DE)
(72) Erfinder: ERLEN, Christoph, 34132 Kassel (DE)
(74) Vertreter: Winter, Brandl - Partnerschaft mbB
(86) Internationale Anmeldenummer: PCT/EP2018/063385
(87) Internationale Veröffentlichungsnummer: WO 2018/215465

(56) Entgegenhaltungen:
- WO-A1-93/10834
- WO-A2-2007/033025
- JP-A- H03 205 060
- US-A1- 2012 238 991
- US-A1- 2013 281 965
- US-A1- 2016 007 901

## Beschreibung

Die Erfindung betrifft eine, insbesondere nach dem Linearperistaltikprinzip arbeitende, volumetrische Pumpe zur kontinuierlichen intravenösen Verabreichung von Infusionen mit einem Sensorsystem zur Erfassung einer Klappenstellung.

Volumetrische Pumpen (auch als Dosierpumpen oder Schlauchpumpen bzw. Infusomaten bekannt), wie beispielsweise eine Infusionspumpe, sind elektrische Geräte für die kontinuierliche intravenöse Gabe von Infusionslösungen. Bekannt sind beispielsweise Infusionspumpen mit einer elektronisch gesteuerten peristaltischen Pumpe an einem Infusionsschlauch, die eine exakte Dosierung der Infusionsflüssigkeit pro Zeiteinheit ermöglichen.

Das Förderprinzip bei Pumpen der genannten Art, die auch als volumengesteuerte bzw. volumetrische Pumpen bezeichnet werden, beruht auf dem System der Schieberperistaltik, mit der ein gleichmäßiger Fluss mit geringer Pulsation und variabler Stärke erzeugt werden kann. Eine Wellenumdrehung der Peristaltik entspricht dabei einem Arbeitsschritt, in dem durch die Kompression eines definierten Schlauchvolumens eine bestimmte Menge Infusionslösung dem Infusionssystem zugeführt wird.

Vorwiegend kommt eine Linearperistaltik zur Anwendung, bei der ein Schlauch mittels einer beispielsweise vorderseitig am Gerät zugänglichen und bedienbaren Klappe bzw. Frontklappe an zumindest einen, sich bewegenden Peristaltikfinger gedrückt wird. Dabei soll ein unkontrollierter Fluss unterbunden werden, und die Flüssigkeitsförderung durch die Bewegung der Peristaltikfinger soll kontrollierbar sein. Für die Funktion des Geräts ist deshalb die Stellung der Klappe, von entscheidender Bedeutung. Vereinfacht kann eine Klappe beispielsweise die Stellungen OFFEN, HALBGESCHLOSSEN und GESCHLOSSEN einnehmen.

Der Zustand OFFEN bedeutet, dass die Peristaltik nicht okklusiv ist, d.h. ein unkontrollierter Fluss durch die Peristaltik nicht unterbunden wird. Der Zustand GESCHLOSSEN bedeutet, dass kein unkontrollierter Fluss auftreten kann, weil in dieser Stellung der Frontklappe die Frontklappe den Schlauch gegen die Peristaltik abklemmt und nur die Bewegung der Peristaltikfinger durch die gesteuerte Peristaltik einen Fluss einer zu fördernden Flüssigkeit bewirken kann.

Bekannte Sensorsysteme erkennen die Frontklappenstellungen OFFEN und GESCHLOSSEN mittels eines oder mehrerer Sensoren und umfassen Näherungsschalter mit binärer Schaltlogik, welche vorwiegend mit elektromechanischen Schaltern und Magnetschaltern ausgeführt sind.

Die Druckschrift WO 931 08 34 A1 zeigt eine nach dem Linearperistaltikprinzip arbeitende, volumetrische Pumpe, deren Frontklappe nicht zur Okkludierung beiträgt. Die Okkludierung wird allein durch einen Peristaltikfinger bewirkt, der den Schlauch an einer gehäusefesten Nut okkludiert. Die Stellung des Peristaltikfingers ist bezüglich seiner Offenstellung und seiner Okkludierungsstellung sensorüberwacht.

Während einem Anwender die Bedeutungen des offenen bzw. geschlossenen Zustands der Klappe bekannt sind und er daher von einer jeweils entsprechenden und für den Patienten sicheren Funktion der Pumpe ausgehen kann, ist der Zustand HALBGESCHLOSSEN für die Sicherheit des Patienten kritisch, da die Pumpe in diesem Zustand den Fluss eventuell nicht kontrollieren kann. Es ist daher zu vermeiden, dass ein Anwender die Frontklappe während des Betriebs der Pumpe bzw. während deren Bedienung in diesem Zustand verharren lässt.

Bisher bekannte Sensorsysteme sind nicht in der Lage, die Klappenstellung HALBGESCHLOSSEN zuverlässig zu erkennen, so dass ein das bekannte Sensorsystem aufweisendes Gerät auf diesen kritischen Zustand nicht reagieren kann.

Vor diesem Hintergrund liegt der Erfindung als eine Aufgabe zugrunde, eine volumetrische Pumpe mit einem Sensorsystem bereitzustellen, das die Stellung einer Frontklappe der volumetrischen Pumpe in zumindest einem offenen Zustand, einem halb geschlossenen Zustand und einem geschlossenen Zustand erkennt.

Diese Funktion soll mit kleinstmöglichem Einsatz von Hardware bzw. Aufwand und geringstmöglichem Kalibrierungsaufwand realisierbar sein.

Darüber hinaus soll das Sensorsystem der volumetrischen Pumpe in der Lage sein, einen Alarm auszugeben, wenn die Frontklappe beispielsweise länger als eine vorbestimmte Zeitdauer in einem halb geschlossenen Zustand verbleibt.

Erfindungsgemäß wird diese Aufgabe durch eine volumetrische Pumpe mit den Merkmalen des Anspruchs 1 gelöst. Vorteilhafte Weiterbildungen der Erfindung sind Gegenstand der beigefügten Unteransprüche.

Gemäß einer zugrunde liegenden erfinderischen Idee ist ein Sensorsystem, das dazu angeordnet ist, zumindest Stellungen einer stirnseitig angeordneten Klappe (Frontklappenstellungen) OFFEN, HALBGESCHLOSSEN und GESCHLOSSEN einer Frontklappe einer volumetrischen Pumpe für insbesondere ein medizinisches Gerät aufzulösen bzw. sicher zu erkennen, mittels eines analogen, eine Rotationsstellung der Frontklappe und eines Klappenhebels auflösenden Messsystems und dynamischer Auswertung des Messsignals realisiert. Vorzugsweise ist die Frontklappe stirnseitig angeordnet. Vorzugsweise weist das Messsystem dazu einen analogen Sensor, beispielsweise einen Hallsensor auf.

Vorzugsweise weist eine Frontklappe einer volumetrischen Pumpe einen manuell zu bedienenden Verschlusshebel und einen durch den Verschlusshebel gesteuerten Verschlussmechanismus auf. Ein Zustand HALBGESCHLOSSEN, d.h. ein zwischen einer Schließstellung und einer Öffnungsstellung liegender halb geschlossener Zustand der Klappe, ist ein Zustand, in dem die Klappe bereits in den Verschlussmechanismus eingreift, der Verschlusshebel jedoch noch nicht vollständig in eine Endposition bewegt ist und daher der Verschlussmechanismus noch nicht vollständig geschlossen ist.

In der Stellung bzw. dem Zustand OFFEN der Klappe befindet sich die Klappe derart außerhalb eines vorbestimmten Erfassungsbereichs des analogen Sensors, dass die Klappe für den Sensor nicht sichtbar ist und der Sensor somit ein Nullsignal liefert. In der Stellung bzw. dem Zustand GESCHLOSSEN der Klappe befindet sich die Klappe derart nahe an dem analogen Sensor und ist die Klappe für den Sensor als sehr nahe liegend sichtbar, wodurch Sensor somit ein starkes oder hohes Signal liefert. Die Stellung HALBGESCHLOSSEN ist in dieser Konfiguration dadurch erkennbar, dass der Sensor die Klappe in einer zwar an die Peristaltik der Pumpe angenäherten Position, jedoch noch nicht vollständig in einer dem geschlossenen Zustand entsprechenden Endlage sieht, und somit ein schwaches Signal liefert, das stärker ist als das dem offenen Zustand entsprechende Nullsignal, aber schwächer ist als das dem geschlossenen Zustand entsprechende starke Signal.

Die vorgenannten unterscheidbaren Signalwerte bzw. Signalpegel werden vorzugsweise durch beispielsweise einen analogen Hallsensor, der in einem Hauptgehäuse der volumetrischen Pumpe angeordnet ist, und einen Magneten, der in der Frontklappe angeordnet ist, realisiert werden. In einer solchen Konfiguration erfasst der Sensor in der Stellung OFFEN der Klappe den Magneten nicht und gibt dementsprechend kein Signal bzw. ein Nullsignal aus, erfasst der Sensor in der Stellung HALBGESCHLOSSEN der Klappe den Magneten an einer entfernten Position und gibt dementsprechend ein schwaches Signal aus, und erfasst der Sensor in der Stellung GESCHLOSSEN der Klappe den Magneten an einer nahen Position und gibt dementsprechend ein starkes Signal aus.

Bevorzugt ist die Erfassung durch den Sensor bzw. das analoge Signal nicht nur mit der Rotationsstellung der Frontklappe verbunden, sondern zusätzlich auch an die Stellung des Verschlusshebels gekoppelt. In diesem Fall wird erst dann, wenn die Klappe geschlossen ist und sich der Verschlusshebel in seiner vorgesehenen und der geschlossenen Stellung der Klappe entsprechenden Endlage befindet, der zuvor erfasste Zustand HALBGESCHLOSSEN aufgehoben, d.h. verlassen, und auf einen nicht mehr kritischen Zustand geschlossen.

Im Falle der vorstehend beschriebenen Paarung aus Hallsensor und Magnet kann dazu beispielsweise der Magnet an dem Bedienhebel oder einen von dem Klappenhebel mechanisch in seiner Position kontrollierten Schließriegel angebracht sein. Eine Auslegung kann derart sein, dass das analoge Sensorsignal z.B. von stark bei offener Klappe nach schwach bei halbgeschlossener Klappe und nach stark bei komplett geschlossener bzw. durch Betätigen des Bedienhebels verriegelter Klappe wechselt, wobei sich der mit dem Bedienhebel gekoppelte Magnet durch den Verriegelungsvorgang des Bedienhebels wieder ein Stück weit vom Sensor bei geschlossener Klappe wegbewegt.

Ferner kann ein Zeitverhalten des analogen Signals zur Plausibilisierung bzw. Absicherung der Signalauswertung genutzt werden. Beispielsweise kann der Sensor dazu angeordnet sein, auf der Grundlage eines Zeitverhaltens die Position oder Stellung der Klappe basierend auf einem Abgleich der Signaldynamik mit einem Modell des Zeitverhaltens auszuwerten. In diesem Fall kann eine Auswertung mittels zu kalibrierender Schwellwerte vorteilhaft entfallen.

Wie vorstehend beschrieben wurde, sind mittels dem erfindungsgemäßen Sensorsystem zumindest drei Klappenstellungen OFFEN, HALBGESCHLOSSEN und GESCHLOSSEN erkennbar und erfassbar. Aufgrund der analogen Auslegung ist es vorteilhaft, dass lediglich ein einzelner Sensor zur Erkennung dieser zumindest drei Zustände erforderlich ist. Alternativ, aber nicht als Bestandteil der Erfindung, ist eine Lösung mit binär arbeitenden Sensoren bzw. Binärsensoren denkbar, in welchem Fall zumindest zwei Sensoren für die Unterscheidung von HALBGESCHLOSSEN und GESCHLOSSEN anzuordnen sind. Die Auswertung des dynamischen Zeitverhaltens ermöglicht vorteilhaft den Verzicht auf die Kalibrierung und damit einen signifikant verringerten Kalibrierungsaufwand. Die Auswertung des dynamischen Zeitverhaltens ermöglicht weiter vorteilhaft eine Plausibilisierung der Sensorsignale und im Hinblick auf Aspekte der Sicherheit eine Überwachung der korrekten Sensorfunktion.

Erfindungsgemäß wird die Aufgabe im Einzelnen gelöst durch ein Sensorsystem für eine nach dem Linearperistaltikprinzip arbeitende volumetrische Pumpe, beinhaltend zumindest eine Sensoreinrichtung; zumindest eine Sensorgebereinrichtung; und eine Sensorausgangssignal-Verarbeitungseinrichtung, wobei die Sensorgebereinrichtung dazu angeordnet ist, die Sensoreinrichtung entlang eines vorbestimmten Wegs, auf dem sich eine fest angeordnete Sensoreinrichtung und eine beweglich angeordnete Sensorgebereinrichtung relativ zueinander bewegen, mit einer Erfassungsgröße zu beaufschlagen, die in Abhängigkeit von einer Wegposition variiert; die Sensoreinrichtung dazu konfiguriert ist, auf der Grundlage der variierenden Erfassungsgröße ein einer jeweiligen Wegposition entsprechendes Erfassungssignal auszugeben; und die Sensorausgangssignal-Verarbeitungseinrichtung dazu angeordnet ist, das von der Sensoreinrichtung ausgegebene Erfassungssignal zu empfangen und auf der Grundlage des empfangenen Erfassungssignals zumindest drei Wegpositionen zu unterscheiden und die Sensorgebereinrichtung an einem von einem Verschlusshebel mechanisch in seiner Position kontrollierten Schließriegel angebracht ist. Vorteilhaft kann auf diese Weise eine Stellung einer Klappe einer Infusionspumpe, in der eine Okklusion des eingelegten Schlauchs nicht gesichert ist, detektiert werden.

Die zumindest drei Wegpositionen beinhalten eine geschlossene Position, in welcher ein in die Pumpe eingelegter Schlauchabschnitt okkludiert ist, eine offene Position, in welcher der in die Pumpe eingelegte Schlauch nicht okkludiert ist, und eine Zwischenposition, die sich zwischen der geschlossenen Position und der offenen Position befindet. Vorteilhaft kann auf diese Weise eine Stellung einer Klappe einer Infusionspumpe an einer Zwischenposition, in der eine Okklusion des eingelegten Schlauchs nicht gesichert ist, zwischen dem regulär geschlossenen und dem regulär offenen Zustand der Klappe detektiert werden.

Bevorzugt ist die Sensoreinrichtung in einem Gehäuse der Pumpe angeordnet. Alternativ, nicht entsprechend der Erfindung, ist die Sensoreinrichtung an einer an dem Gehäuse verstellbar angelenkten Wegpositionsänderungseinrichtung angeordnet und ist die Sensorgebereinrichtung in einem Gehäuse der Pumpe angeordnet. Vorteilhaft werden dadurch mannigfaltige Freiheitsgrade für die Anordnung eines Sensors als Sensoreinrichtung und eines Geberelements als Sensorgebereinrichtung sowie die Wahl eines für eine Relativbewegung zwischen denselben und damit für ein sich positions- bzw. wegabhängig ändernden Erfassungssignals sorgenden Bestandteils der Pumpen-Klappen-Anordnung bereitgestellt. Freiheitsgrade der genannten Art sind in den nachfolgenden Aspekten genauer angegeben.

Bevorzugt ist die Wegpositionsänderungseinrichtung eine Klappeneinrichtung der Pumpe, die als eine Verschlusseinrichtung konfiguriert und dazu angeordnet ist, einen in die Pumpe eingelegten Schlauchabschnitt in einem geschlossenen Zustand gegen ein in der Pumpe angeordnetes, Flüssigkeit in dem Schlauchabschnitt förderndes Peristaltikelement zu okkludieren und in einem geöffneten Zustand gegenüber demselben nicht okklusiv zu sein, oder ist die Wegpositionsänderungseinrichtung eine Scharniereinrichtung an der Klappeneinrichtung, die die Klappeneinrichtung verstellbar bzw. klappbar an das Gehäuse anlenkt, oder ist die Wegpositionsänderungseinrichtung ein mit der Klappeneinrichtung der Pumpe beweglich gekoppelter Schließriegel, oder ist die Wegpositionsänderungseinrichtung ein mit der Klappeneinrichtung und/oder dem Gehäuse der Pumpe gekoppelter Verschlusshebel.

Gemäß der Erfindung ist ein Sensorgegenstück (z.B. Magnet) an einem Schließriegel und ein Sensor in der Pumpe angeordnet.

Nach weiteren Beispielen, welche nicht Teil der Erfindung sind, können ein Sensor in der Pumpe und ein Sensorgegenstück (z.B. Magnet) in der beispielsweise in Form einer Frontklappe ausgebildeten Klappeneinrichtung angeordnet sein, können ein Sensor in der Frontklappe und ein Sensorgegenstück in der Pumpe angeordnet sein, können ein Sensor in einem Frontklappenscharnier und ein Sensorgegenstück in der Pumpe angeordnet sein, können ein Sensor in einem beweglichen Schließriegel und ein Sensorgegenstück in der Pumpe angeordnet sein, können ein Sensor in einem Verschlusshebel und ein Sensorgegenstück in der Pumpe angeordnet sein, können ein Sensorgegenstück (z.B. Magnet) an einem Verschlusshebel und ein Sensor in der Pumpe angeordnet sein, und/oder können ein Sensorgegenstück (z.B. Magnet) unbeweglich an einer Frontklappe und ein Sensor in der Pumpe angeordnet sein.

Bevorzugt ist das Erfassungssignal der Sensoreinrichtung an eine eine erste Wegposition bildende Rotationsstellung der Klappeneinrichtung und an eine eine zweite Wegposition bildende Stellung des Verschlusshebels gekoppelt, und ist die Sensorausgangssignal-Verarbeitungseinrichtung dazu konfiguriert, nur dann, wenn die erste Wegposition anzeigt, dass die Frontklappe vollständig geschlossen ist, und die zweite Wegposition anzeigt, dass sich der Verschlusshebel in einer vorgesehenen und der geschlossenen Stellung der Klappeneinrichtung entsprechenden Endlage befindet, ein zuvor erfasster Zustand einer Zwischenposition aufgehoben wird.

Bevorzugt ist die Sensoreinrichtung ein Hallsensor und ist die Sensorgebereinrichtung ein den Hallsensor beaufschlagender Magnet, oder ist die Sensoreinrichtung ein kapazitiver Sensor und ist die Sensorgebereinrichtung dazu angeordnet, in der Sensoreinrichtung eine einem zurückgelegten Weg der Verschlussvorrichtung entsprechende Änderung einer Kapazität herbeizuführen, oder ist die Sensoreinrichtung ein induktiver Sensor und ist die Sensorgebereinrichtung dazu angeordnet, in der Sensoreinrichtung eine einem zurückgelegten Weg der Verschlussvorrichtung entsprechende Änderung eines Magnetfelds herbeizuführen, oder ist die Sensoreinrichtung ein optischer Sensor und ist die Sensorgebereinrichtung dazu angeordnet, an der Sensoreinrichtung eine einem zurückgelegten Weg der Verschlussvorrichtung entsprechende Änderung eines Lichteinfalls herbeizuführen, oder ist die Sensoreinrichtung ein Element mit veränderbarem Widerstand, vorzugsweise nach Art eines Potentiometers, und ist die Sensorgebereinrichtung dazu angeordnet, an der Sensoreinrichtung eine einem zurückgelegten Weg der Verschlussvorrichtung entsprechende Widerstandsänderung herbeizuführen.

Beispielsweise können vorteilhaft eine Ausführung mittels analoger Hallsensoren, eine Ausführung mittels kapazitiver oder induktiver Sensoren, eine Ausführung mittels optischer Sensoren, und/oder eine Ausführung mittels Bauelementen mit veränderbaren Widerständen wie z.B. eines Potentiometers und dergleichen vorgesehen sein.

Bevorzugt erfasst die Sensoreinrichtung in einer ersten Wegposition, die einem nicht okkludierten Offenzustand entspricht, das Vorhandensein der Sensorgebereinrichtung nicht und gibt ein Nullsignal als ein erstes Erfassungssignal an die Sensorausgangssignal-Verarbeitungseinrichtung aus; erfasst die Sensoreinrichtung in einer dritten Wegposition, die einem okkludierten Geschlossenzustand entspricht, ein nahes Vorhandensein der Sensorgebereinrichtung anhand einer starken Beaufschlagung mit der Erfassungsgröße und gibt ein der starken Beaufschlagung entsprechend starkes Sensorausgangssignal als ein drittes Erfassungssignal an die Sensorausgangssignal-Verarbeitungseinrichtung aus; und erfasst die Sensoreinrichtung in einer zweiten Wegposition, die einem Zwischenzustand zwischen dem okkludierten Geschlossenzustand und dem nicht okkludierten Offenzustand entspricht, ein fernes Vorhandensein der Sensorgebereinrichtung anhand einer gegenüber der starken Beaufschlagung schwächeren, jedoch oberhalb des Nullsignals liegenden Beaufschlagung mit der Erfassungsgröße und gibt ein der schwächeren Beaufschlagung entsprechend schwächeres Sensorausgangssignal als ein zweites Erfassungssignal an die Sensorausgangssignal-Verarbeitungseinrichtung aus.

Bevorzugt ist die Sensorausgangssignal-Verarbeitungseinrichtung dazu angeordnet, das erste, das zweite und/oder das dritte Erfassungssignal entlang des vorbestimmten Wegs als analoge Signale dynamisch auszuwerten.

Bevorzugt ist die Sensorausgangssignal-Verarbeitungseinrichtung dazu angeordnet, auf der Grundlage eines Zeitverhaltens des Erfassungssignals eine Plausibilisierung des Erfassungssignals durchzuführen.

Bevorzugt ist die Sensorausgangssignal-Verarbeitungseinrichtung dazu konfiguriert, die Wegposition durch einen Abgleich der Dynamik des Erfassungssignals mit einem Modell des Zeitverhaltens auszuwerten.

Bevorzugt ist die Sensorausgangssignal-Verarbeitungseinrichtung dazu konfiguriert, ein Alarmsignal auszugeben, wenn die_Zwischenposition, die sich zwischen der geschlossenen Position und der offenen Position befindet, länger als eine vorbestimmte Zeitdauer erfasst wird.

Die Einrichtungen, Strukturen, Konfigurationen und/oder Komponenten, die das hierin beschriebene Sensorsystem bilden, können dazu konfiguriert sein, eine Vielzahl von Modifikationen bereitzustellen, einschließlich mehr oder weniger vorinstallierter Konfigurationen und/oder an geeigneter Stelle vorzusehender und bei Verwendung/in Betrieb verbundener oder angeschlossener separater Teile.

Die Erfindung wird nachstehend unter Bezugnahme auf die schematisch und vereinfacht bereitgestellte, beigefügte Zeichnung näher beschrieben. Es zeigen:
Fig. 1 eine Frontansicht einer volumetrischen Pumpe, beispielsweise einer Infusionspumpe, die ein Sensorsystem gemäß einem Ausführungsbeispiel beinhaltet; und
Fig. 2 eine Seitenansicht der volumetrischen Pumpe nach Fig. 1

Ähnliche oder gleiche Teile, die in der Zeichnung dargestellt sind, können durch gleiche Bezugszeichen oder nicht durch Bezugszeichen bezeichnet sein, und Einzelheiten derselben werden nicht redundant beschrieben oder erklärt. Außerdem können über die Zeichnung hinweg Abschnitte und/oder Komponenten ohne unmittelbare Bedeutung für die Beschreibung der Erfindung weggelassen oder verborgen sein, um eine verbesserte Sichtbarkeit wichtigerer Teile zu schaffen.

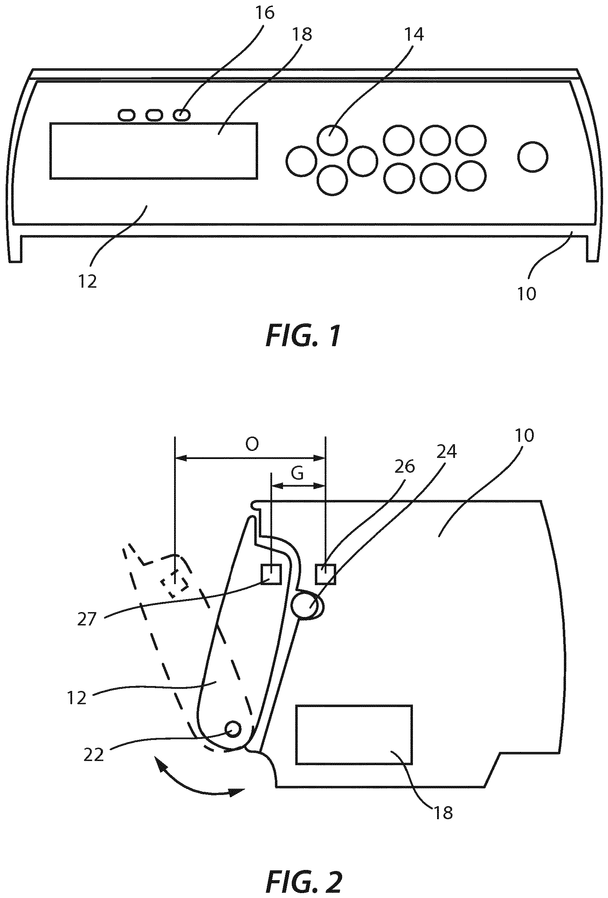

Bei der in Fig. 1 dargestellten Stirn- oder Frontansicht volumetrischen Pumpe, die ein Sensorsystem gemäß einem Ausführungsbeispiel beinhaltet, sind in einem Gehäuse 10 der Pumpe die für die grundlegende Funktion der Pumpe erforderlichen Funktionseinheiten, Bauteile und Komponenten aufgenommen. Diese sind an sich bekannt und werden daher hier nicht redundant beschrieben. Eine stirn- oder frontseitige Klappe (Frontklappe) 12 ist klappbar an dem Gehäuse 10 angelenkt. Die Klappe 12 erstreckt sich vorzugsweise über im Wesentlichen die gesamte Breite des Pumpengehäuses, da eine ihrer Aufgaben darin besteht, durch geeignetes Auf- bzw. Wegklappen einen Zugang für das Einlegen einer Flüssigkeit, beispielsweise eine Infusionslösung, transportierenden Schlauchs oder Schlauchabschnitts, in die Pumpe zu eröffnen. Außenseitig an der Klappe sind Bedienelemente 14 (angedeutet durch Kreise, nur eines davon ist mit einem Bezugszeichen bezeichnet), zumindest einer Betriebszustand-Signalisierungsanzeige 16 (angedeutet durch abgerundete Rechtecke, nur eines davon ist mit einem Bezugszeichen bezeichnet), die beispielsweise als Leuchtmittel bereitgestellt sind, und eine Anzeigeeinrichtung 18, die beispielsweise als Flüssigkristallanzeige ausgeführt sein kann, für beispielsweise eine Programmführung und Benutzerkommunikation angeordnet.

Fig. 2 zeigt eine Seitenansicht der in Fig. 1 dargestellten volumetrischen Pumpe. Die Klappe 12 ist an einer Gelenk- oder Scharniereinrichtung 22 (Frontklappenscharnier) im unteren Bereich des Gehäuses 10 um eine in Längsrichtung des Gehäuses 10 verlaufende Achse gegenüber dem Gehäuse 10 entlang eines vorbestimmten Wegs klappbar oder verschwenkbar.

Die Klappe 12 kann dabei entlang des vorbestimmten Wegs eine Anzahl von Wegpositionen einnehmen.

Eine erste Stellung bzw. Wegposition ist eine Schließposition oder Geschlossenposition, in der die Klappe 12 nahe an das Gehäuse 10 geführt ist und dafür sorgt, dass ein in die Pumpe in beispielsweise Schlauchführungsabschnitte und/oder Schlauchaufnahmen eingelegter Schlauch oder Schlauchabschnitt 24 gegen eine (nicht gezeigte) Peristaltik (Peristaltikfinger, Peristaltikelement) presst und den Schlauch 24 okkludiert.

Eine zweite Stellung bzw. Wegposition ist eine Offenposition, in der die Klappe 12 von dem Gehäuse weggeklappt ist (in Fig. 2 in durchbrochener Linie dargestellt) und den Zugang zur Pumpe freigibt. Ein eingelegter Schlauch 24 wird in der Offenposition der Klappe 12 nicht okkludiert.

Eine dritte Stellung bzw. Wegposition ist eine Zwischenposition zwischen der Geschlossenposition bzw. ersten Wegposition und der Offenposition. Da eine von der Klappe 12 übertragene Okklusionskraft in der Regel durch beispielsweise einen beweglichen Schließriegel und/oder einen Verschlusshebel (beide nicht gezeigt) erzeugt und gehalten, zumindest aber unterstützt wird, kann dann, wenn sich der Schließriegel und/oder der Verschlusshebel an einer Zwischenposition nicht in einer vorbestimmten Endposition befindet, eine nicht vollständige Okklusion des Schlauchs 24 auftreten.

In anderen Worten kann ein Anwender zwar die Klappe 12 schließen und den Schließriegel bzw. den Verschlusshebel betätigen. Sofern dies unbemerkt unvollständig erfolgt, kann dennoch die Klappe 12 in einer Zwischenposition (die hierin auch als ein halb geschlossener Zustand bezeichnet wird bzw. einem solchen Zustand entspricht) verbleiben und nicht ausreichend okkludieren, obwohl der Anwender davon ausgeht, dass ein ordnungsgemäßer Schließvorgang durchgeführt wurde.

Während einem Anwender die Bedeutungen des offenen bzw. geschlossenen Zustands der Klappe bekannt sind und er daher von einer jeweils entsprechenden und für den Patienten sicheren Funktion der Pumpe ausgehen kann, ist der halb geschlossene Zwischenzustand für die Sicherheit des Patienten kritisch, da die Pumpe in diesem Zustand den durch den Schlauch oder Schlauchabschnitt 24 geförderten Fluss nicht sicher kontrollieren kann. Der Anwender darf daher die Klappe 12 während des Betriebs der Pumpe bzw. während deren Bedienung nicht in diesem Zustand belassen.

Zur Detektion bzw. Erfassung eines wie vorstehenden Zwischenzustands, d.h. einer Stellung der Klappe 12 zwischen der Offenposition und der Geschlossenposition, sind gemäß dem vorliegenden Ausführungsbeispiel zumindest ein Sensor 26 (Sensoreinrichtung) und zumindest ein den Sensor mit einer Erfassungsgröße beaufschlagendes Geberelement 27 (Sensorgebereinrichtung) in dem Gehäuse und in der Klappe 12 derart zueinander zugeordnet/angeordnet, dass in Übereinstimmung mit der Entfernung zwischen dem Sensor 16 und dem Geberelement 27 zumindest der Geschlossenzustand, der Offenzustand und eine Zwischenposition zwischen dem Geschlossenzustand und dem Offenzustand, d.h. erste, die zweite und die dritte Wegposition der Klappe 12, voneinander unterscheidbar detektierbar sind.

Im Einzelnen weist das Sensorsystem gemäß dem Ausführungsbeispiel den zumindest einen Sensor 26; das zumindest eine Geberelement 27 und im Weiteren beispielsweise einen Mikrocontroller oder Mikroprozessor 28 mit geeigneter Speicherausstattung und Peripherie (Sensorausgangssignal-Verarbeitungseinrichtung), der/die als solche bekannt ist/sind und daher nicht redundant beschrieben wird/werden, auf. Der Sensor 26 kann fest in dem Gehäuse 10 und das Geberelement 27 in der Klappe 12 und damit zusammen mit dieser beweglich angeordnet sein, wie in Fig. 2 dargestellt. Alternativ kann das Geberelement 27 fest in dem Gehäuse 10 und der Sensor 26 in der Klappe 12 und zusammen mit dieser beweglich angeordnet sein.

Grundsätzlich besteht keine besondere Beschränkung bezüglich der Anordnungsposition von Sensor 26 und Geberelement 27, solange das Geberelement 27 den Sensor 26 entlang eines vorbestimmten Wegs, auf dem sich ein fest angeordneter Sensor 26 und ein beweglich angeordnetes Geberelement 27 oder ein beweglich angeordneter Sensor 26 und ein fest angeordnetes Geberelement 27 relativ zueinander bewegen können und dabei das Geberelement 27 den Sensor 26 mit einer Erfassungsgröße beaufschlagen kann, die sich in Abhängigkeit von einer Wegposition, d.h. der ersten, der zweiten und der dritten Wegposition wie vorstehend beschrieben, ändert.

Dann nämlich kann der Sensor 26 auf der Grundlage der sich wegabhängig ändernden Erfassungsgröße ein einer jeweiligen Wegposition entsprechendes, in dem vorliegenden Ausführungsbeispiel analoges, Erfassungssignal an den Mikrocontroller oder Mikroprozessor 28 ausgeben.

Der Mikrocontroller oder Mikroprozessor 28 ist sodann dazu angeordnet, das von dem Sensor 26 ausgegebene Erfassungssignal zu empfangen, auszuwerten und im Ergebnis zumindest drei Wegpositionen, d.h. die erste, die zweite und die dritte Wegposition, die jeweils die geschlossene Position, in welcher ein in die Pumpe eingelegter Schlauch oder Schlauchabschnitt 24 okkludiert ist, die offene Position, in welcher der in die Pumpe eingelegte Schlauch oder Schlauchabschnitt 24 nicht okkludiert ist, und die Zwischenposition zwischen der geschlossenen Position und der offenen Position anzeigen.

Es wird angemerkt, dass die Zwischenposition zwar grundlegend eine beliebige Position zwischen der offenen Position bzw. Offenposition und der geschlossenen Position bzw. Geschlossenposition sein kann, zweckmäßig aber aus einem solchen Bereich möglicher Zwischenpositionen zumindest eine vorbestimmte Zwischenposition, ab welcher ein Geschlossenzustand nicht mehr und/oder ein Offenzustand noch nicht erfasst wird, grenzbildend über z.B. eine(n) Schwellenwertbetrachtung (Vergleich) ausgewählt wird. Da in dem vorliegenden Ausführungsbeispiel die Erfassung unter Verwendung eines analogen Signals erfolgt, sind entsprechende Schwellenwerte wahlfrei festlegbar, und der Mikrocontroller oder Mikroprozessor 28 kann dazu ausgelegt sein, eine wahlfreie Anzahl von Zwischenpositionen zu verarbeiten und zu detektieren.

Erfasst der Mikrocontroller oder Mikroprozessor 28 eine Zwischenposition, die sich vorbestimmt zwischen der geschlossenen Position und der offenen Position befindet, kann er dazu konfiguriert sein, ein Alarmsignal über beispielsweise die Betriebszustand-Signalisierungsanzeige 16, die Anzeigeeinrichtung 18 und/oder ein Tonsignal aus einem (nicht gezeigten) Lautsprecher auszugeben, um den Anwender über diesen Zustand in Kenntnis zu setzen, und/oder den Betrieb der Pumpe in geeigneter Weise einzuschränken, beispielsweise anzuhalten. Das Alarmsignal kann verzögert ausgegeben werden, beispielsweise erst nachdem die Zwischenposition länger als eine vorbestimmte Zeitdauer erfasst worden ist.

Im vorstehenden Zusammenhang bildet die Klappe 12 somit eine an dem Gehäuse 10 verstellbar angelenkte Wegpositionsänderungseinrichtung und gleichzeitig eine Verschlusseinrichtung, um einen in die Pumpe eingelegten Schlauch oder Schlauchabschnitt 24 in dem geschlossenen Zustand gegen das, in der Pumpe angeordnete Flüssigkeit, wie beispielsweise eine Infusionslösung, in dem Schlauch oder Schlauchabschnitt 24 fördernde Peristaltikelement zu okkludieren und um in dem geöffneten Zustand gegenüber dem Peristaltikelement nicht okklusiv zu sein.

In jeweils alternativen Ausführungsformen kann die Wegpositionsänderungseinrichtung das Scharnier 22 der Klappe 12, ein mit der Klappe 12 der Pumpe beweglich gekoppelter Schließriegel, oder ein mit der Klappe 12 und/oder dem Gehäuse 10 der Pumpe gekoppelter Verschlusshebel sein.

Gemäß der Erfindung ist das Geberelement 27 als ein Sensorgegenstück wie z.B. ein Magnet an dem Schließriegel und der Sensor 26 in der Pumpe angeordnet. Nach Beispielen, welche nicht Teil der Erfindung sind, können der Sensor 26 in der Pumpe und das Geberelement 27 als ein Sensorgegenstück wie z.B. ein Magnet in der Klappe 12 angeordnet sein, können der Sensor 26 in der Klappe 12 und das Geberelement 27 in der Pumpe angeordnet sein, können der Sensor 26 in dem Scharnier 22 der Klappe 12 angeordnet und das Geberelement 27 in der Pumpe angeordnet sein, können der Sensor 26 in dem beweglichen Schließriegel und das Geberelement 27 in der Pumpe angeordnet sein, können der Sensor 26 in dem Verschlusshebel und das Geberelement 27 in der Pumpe angeordnet sein, können das Geberelement 27 als ein Sensorgegenstück wie z.B. ein Magnet an dem Verschlusshebel und der Sensor 26 in der Pumpe angeordnet sein, und/oder können das Geberelement 27 als ein Sensorgegenstück wie z.B. ein Magnet unbeweglich an der Klappe 12 und der Sensor 26 in der Pumpe angeordnet sein.

Vorteilhaft kann außerdem das Erfassungssignal des Sensors 26 sowohl an eine Stellung der Klappe 12, die als eine erste Wegposition eine Klappenwegposition bildet, als auch an eine Stellung des Verschlusshebels, die als eine zweite Wegposition eine Verschlusshebelwegposition bildet gekoppelt sein. In diesem Fall kann der Mikrocontroller oder Mikroprozessor 28 dazu konfiguriert sein, nur dann zu bestimmen, dass ein zuvor erfasster Zustand einer Zwischenposition aufgehoben ist, wenn die Klappenwegposition anzeigt, dass die Klappe 12 vordefiniert geschlossen ist und die Verschlusshebelwegposition anzeigt, dass sich der Verschlusshebel in einer vorgesehenen und beispielsweise der geschlossenen Stellung der Klappe 12 entsprechenden Endlage befindet.

Der Sensor 26 kann ein Hallsensor und das Geberelement 27 kann ein den Hallsensor beaufschlagender Magnet sein. In jeweils alternativen Ausführungsformen kann der Sensor 26 ein kapazitiver Sensor sein und kann das Geberelement 27 den Sensor 26 mit einer einem zurückgelegten Weg der Klappe 12 entsprechenden Kapazitätsänderung zu beaufschlagen, oder kann der Sensor 26 ein induktiver Sensor sein und kann das Geberelement 27 den Sensor 26 mit einer einem zurückgelegten Weg der Klappe 12 entsprechenden Magnetfeldänderung beaufschlagen, oder kann der Sensor 26 ein optischer Sensor sein und kann das Geberelement 27 an dem Sensor 26 eine einem zurückgelegten Weg der Verschlussvorrichtung entsprechende Änderung eines Lichteinfalls bzw. einer Menge einfallenden Lichts herbeiführen, oder kann der Sensor 26 ein Element mit veränderbarem Widerstand, vorzugsweise nach Art eines Potentiometers, sein und kann das Geberelement 27 an dem Sensor 26 eine einem zurückgelegten Weg der Verschlussvorrichtung entsprechende Widerstandsänderung einsteuern.

Das Sensorsystem in dem vorliegenden Ausführungsbeispiel nutzt analoge Signale, beispielsweise von Hallsensoren, kapazitiven oder induktiven Sensoren, optischen Sensoren und/oder Bauelementen mit veränderbaren Widerständen wie z.B. einem Potentiometer und dergleichen.

Es besteht jedoch keine Beschränkung hierauf. Das Sensorsystem ist gleichfalls auf der Grundlage binärer Signale bzw. Binärsignale liefernder Sensoren darstellbar. In diesem Fall erhöht sich die Anzahl der wenigstens anzuordnenden Sensoren entsprechend der zu unterscheidenden Wegpositionen. Im Falle dreier auf binärer Grundlage zu unterscheidender Wegpositionen sind dann beispielsweise wenigstens zwei Binärsignale ausgebende Sensoren 26 vorzusehen.

Die Erfassung der einzelnen Wegpositionen erfolgt in dem vorliegenden Ausführungsbeispiel wie nachstehend beschrieben. In bzw. an einer ersten Wegposition der Klappe 12, die dem nicht okkludierten Offenzustand (Entfernung O zwischen Sensor 26 und Geberelement 27 in Fig. 2) entspricht, sieht bzw. erfasst der Sensor 26 das Vorhandensein des Geberelements 27 nicht und gibt somit ein Nullsignal als ein erstes Erfassungssignal an den Mikrocontroller oder Mikrocomputer 28 aus. An einer dritten Wegposition der Klappe 12, die dem okkludierten Geschlossenzustand entspricht (Entfernung G zwischen Sensor 26 und Geberelement 27 in Fig. 2), sieht bzw. erfasst der Sensor 26 das Geberelement 27 aufgrund der ihn stark beaufschlagenden Erfassungsgröße in kurzer Entfernung bzw. nahe vor sich, und gibt daher ein der starken Beaufschlagung entsprechendes, starkes Sensorausgangssignal als ein drittes Erfassungssignal an den Mikrocontroller oder Mikrocomputer 28 aus. An einer zweiten Wegposition, die einem Zwischenzustand zwischen dem okkludierten Geschlossenzustand und dem nicht okkludierten Offenzustand entspricht, sieht bzw. erfasst der Sensor 26 aufgrund der ihn gegenüber der starken Beaufschlagung an der dritten Wegposition schwächer, jedoch mit einer Stärke oberhalb des Nullsignals beaufschlagenden Erfassungsgröße das Geberelement 27 entfernter als an der dritten Wegposition und gibt ein der schwächeren Beaufschlagung entsprechend schwächeres Sensorausgangssignal als ein zweites Erfassungssignal an den Mikrocontroller oder Mikrocomputer 28 aus.

Das erste, das zweite und/oder das dritte Erfassungssignal können sodann von dem Mikrocontroller oder Mikrocomputer 28 entlang des vorbestimmten Wegs der Klappe 12 als analoge Signale dynamisch ausgewertet werden. Beispielsweise kann der Mikrocontroller oder Mikrocomputer 28 auf der Grundlage eines Zeitverhaltens eines oder aller der Erfassungssignale eine Plausibilisierung des Erfassungssignals durchführen, um das Sensorsystem auf korrekte und sichere Funktion zu überwachen. Die Wegposition kann beispielsweise durch einen Abgleich der Dynamik des Erfassungssignals mit einem Modell des Zeitverhaltens ausgewertet werden.

Obwohl bestimmte Größen, wie beispielsweise Gewicht, absolute Länge, Breite und Dicke, Farbgebung, Form und unwesentliche Einzelheiten nicht gezeigt sind, liegen derartige Spezifikationen für den Fachmann ersichtlich im Rahmen der vorstehend beschriebenen Ausführungsform und Erfindung. Darüber hinaus versteht sich, dass der konkrete Text, eine Abfolge und ein Inhalt von in der Zeichnung gezeigten und hierin beschriebenen Konfigurationen und Komponenten lediglich darstellender Natur und beispielhaft sind, und dass die Vorrichtung und deren Betriebsablauf nicht auf diese beschränkt sind.

## Patentansprüche

1. Volumetrische Pumpe mit einem Gehäuse (10),
einer Frontklappe (12), welche einen manuell zu bedienenden Verschlusshebel hat, und
einem Sensorsystem mit berührungslosen Sensoren für die nach dem Linearperistaltikprinzip arbeitende volumetrische Pumpe, welches die Stellung der Frontklappe und/oder des Verschlusshebels zu dem Gehäuse (10) der volumetrischen Pumpe erfasst, wobei das Sensorsystem folgendes aufweist:
zumindest eine Sensoreinrichtung (26);
zumindest eine Sensorgebereinrichtung (27); und
eine Sensorausgangssignal-Verarbeitungseinrichtung (28), wobei
die Sensorgebereinrichtung (27) dazu angeordnet ist, die Sensoreinrichtung (26) entlang eines vorbestimmten Wegs, auf dem sich eine fest, vorzugsweise an dem Gehäuse (10) angeordnete Sensoreinrichtung (26) und eine beweglich angeordnete Sensorgebereinrichtung (27) relativ zueinander bewegen, mit einer Erfassungsgröße zu beaufschlagen, die in Abhängigkeit von einer Wegposition variiert;
die Sensoreinrichtung (26) dazu konfiguriert ist, auf der Grundlage der variierenden Erfassungsgröße ein einer jeweiligen Wegposition entsprechendes Erfassungssignal auszugeben;
die Sensorausgangssignal-Verarbeitungseinrichtung (28) dazu angeordnet und ausgebildet ist, das von der Sensoreinrichtung (26) ausgegebene Erfassungssignal zu empfangen und auf der Grundlage des empfangenen Erfassungssignals zumindest drei Wegpositionen zu unterscheiden, wobei
die zumindest drei Wegpositionen eine geschlossene Position, in welcher ein in die Pumpe eingelegter Schlauchabschnitt (24) okkludiert ist, eine offene Position, in welcher der in die Pumpe eingelegte Schlauchabschnitt (24) nicht okkludiert ist, und eine Zwischenposition, die sich zwischen der geschlossenen Position und der offenen Position befindet, beinhalten; und
die Sensorgebereinrichtung (27) an einem von dem Verschlusshebel mechanisch in seiner Position kontrollierten Schließriegel angebracht ist.

2. Volumetrische Pumpe nach Anspruch 1, wobei bei dem Sensorsystem
die Sensoreinrichtung (26) in einem Gehäuse (10) der Pumpe angeordnet ist und die Sensorgebereinrichtung (27) an einer an dem Gehäuse (10) verstellbar angelenkten Wegpositionsänderungseinrichtung (12; 22) angeordnet ist, oder
die Sensoreinrichtung (26) an einer an dem Gehäuse (10) verstellbar angelenkten Wegpositionsänderungseinrichtung (12; 22) angeordnet ist und die Sensorgebereinrichtung (27) in einem Gehäuse (10) der Pumpe angeordnet ist.

3. Volumetrische Pumpe nach Anspruch 2, wobei bei dem Sensorsystem
die Wegpositionsänderungseinrichtung (12; 22) eine Klappeneinrichtung (12) der Pumpe ist, die als eine Verschlusseinrichtung konfiguriert und dazu angeordnet ist, einen in die Pumpe eingelegten Schlauchabschnitt (24) in einem geschlossenen Zustand gegen ein in der Pumpe angeordnetes, Flüssigkeit in dem Schlauchabschnitt (24) förderndes Peristaltikelement zu okkludieren und in einem geöffneten Zustand gegenüber demselben nicht okklusiv zu sein, oder
die Wegpositionsänderungseinrichtung (12; 22) eine Scharniereinrichtung (22) an der Klappeneinrichtung (12) ist, die die Klappeneinrichtung (12) verstellbar an das Gehäuse (10) anlenkt, oder
die Wegpositionsänderungseinrichtung ein mit der Klappeneinrichtung (12) der Pumpe beweglich gekoppelter Schließriegel ist, oder
die Wegpositionsänderungseinrichtung ein mit der Klappeneinrichtung (12) und/oder dem Gehäuse (10) der Pumpe gekoppelter Verschlusshebel ist.

4. Volumetrische Pumpe nach Anspruch 3, wobei bei dem Sensorsystem
das Erfassungssignal der Sensoreinrichtung (26) an eine eine erste Wegposition bildende Rotationsstellung der Klappeneinrichtung (12) und an eine eine zweite Wegposition bildende Stellung des Verschlusshebels gekoppelt ist, und
die Sensorausgangssignal-Verarbeitungseinrichtung (28) dazu konfiguriert ist, nur dann, wenn die erste Wegposition anzeigt, dass die Klappeneinrichtung (12) vollständig geschlossen ist, und die zweite Wegposition anzeigt, dass sich der Verschlusshebel in einer vorgesehenen und der geschlossenen Stellung der Klappeneinrichtung entsprechenden Endlage befindet, ein zuvor erfasster Zustand einer Zwischenposition aufgehoben wird.

5. Volumetrische Pumpe nach einem der vorangehenden Ansprüche, wobei bei dem Sensorsystem
die Sensoreinrichtung (26) ein Hallsensor ist und die Sensorgebereinrichtung (27) ein den Hallsensor beaufschlagender Magnet ist, oder
die Sensoreinrichtung (26) ein kapazitiver Sensor ist und die Sensorgebereinrichtung (27) dazu angeordnet ist, in der Sensoreinrichtung (26) eine einem zurückgelegten Weg der Verschlussvorrichtung entsprechende Änderung einer Kapazität herbeizuführen, oder
die Sensoreinrichtung (26) ein induktiver Sensor ist und die Sensorgebereinrichtung (27) dazu angeordnet ist, in der Sensoreinrichtung (26) eine einem zurückgelegten Weg der Verschlussvorrichtung entsprechende Änderung eines Magnetfelds herbeizuführen, oder
die Sensoreinrichtung (26) ein optischer Sensor ist und die Sensorgebereinrichtung (27) dazu angeordnet ist, an der Sensoreinrichtung (26) eine einem zurückgelegten Weg der Verschlussvorrichtung entsprechende Änderung eines Lichteinfalls herbeizuführen, oder
die Sensoreinrichtung (26) ein Element mit veränderbarem Widerstand, vorzugsweise nach Art eines Potentiometers, ist und die Sensorgebereinrichtung (27) dazu angeordnet ist, an der Sensoreinrichtung eine einem zurückgelegten Weg der Verschlussvorrichtung entsprechende Widerstandsänderung herbeizuführen.

6. Volumetrische Pumpe nach einem der vorangehenden Ansprüche, wobei bei dem Sensorsystem
die Sensoreinrichtung (26) in einer ersten Wegposition, die einem nicht okkludierten Offenzustand entspricht, das Vorhandensein der Sensorgebereinrichtung (27) nicht erfasst und ein Nullsignal als ein erstes Erfassungssignal an die Sensorausgangssignal-Verarbeitungseinrichtung (28) ausgibt;
die Sensoreinrichtung (26) in einer dritten Wegposition, die einem okkludierten Geschlossenzustand entspricht, ein nahes Vorhandensein der Sensorgebereinrichtung (27) anhand einer starken Beaufschlagung mit der Erfassungsgröße erfasst und ein der starken Beaufschlagung entsprechend starkes Sensorausgangssignal als ein drittes Erfassungssignal an die Sensorausgangssignal-Verarbeitungseinrichtung (28) ausgibt; und
die Sensoreinrichtung (26) in einer zweiten Wegposition, die einem Zwischenzustand zwischen dem okkludierten Geschlossenzustand und dem nicht okkludierten Offenzustand entspricht, ein fernes Vorhandensein der Sensorgebereinrichtung (27) anhand einer gegenüber der starken Beaufschlagung schwächeren, jedoch oberhalb des Nullsignals liegenden Beaufschlagung mit der Erfassungsgröße erfasst und ein der schwächeren Beaufschlagung entsprechend schwächeres Sensorausgangssignal als ein zweites Erfassungssignal an die Sensorausgangssignal-Verarbeitungseinrichtung (28) ausgibt.

7. Volumetrische Pumpe nach Anspruch 6, wobei bei dem Sensorsystem
die Sensorausgangssignal-Verarbeitungseinrichtung (28) dazu angeordnet ist, das erste, das zweite und/oder das dritte Erfassungssignal entlang des vorbestimmten Wegs als analoge Signale dynamisch auszuwerten.

8. Volumetrische Pumpe nach einem der vorangehenden Ansprüche, wobei bei dem Sensorsystem
die Sensorausgangssignal-Verarbeitungseinrichtung (28) dazu angeordnet ist, auf der Grundlage eines Zeitverhaltens des Erfassungssignals eine Plausibilisierung des Erfassungssignals durchzuführen, und/oder
die Sensorausgangssignal-Verarbeitungseinrichtung (28) dazu konfiguriert ist, die Wegposition durch einen Abgleich der Dynamik des Erfassungssignals mit einem Modell des Zeitverhaltens auszuwerten.

9. Volumetrische Pumpe nach einem der Ansprüche 1 bis 8, wobei bei dem Sensorsystem
die Sensorausgangssignal-Verarbeitungseinrichtung (28) dazu konfiguriert ist, ein Alarmsignal auszugeben, wenn die_Zwischenposition, die sich zwischen der geschlossenen Position und der offenen Position befindet, länger als eine vorbestimmte Zeitdauer erfasst wird.

## Claims

1. A volumetric pump including a housing (10),
a front flap (12) having a locking lever to be operated manually, and
a sensor system with contactless sensors for the volumetric pump operating according to the linear peristalsis principle, which detects the position of a front flap and/or the locking lever relative to the housing (10) of the volumetric pump, the sensor system comprising:
at least one sensor unit (26);
at least one sensor transmitter unit (27); and
a sensor output signal processing unit (28), wherein
the sensor transmitter unit (27) is arranged to apply a detection variable varying as a function of a travel position to the sensor unit (26) along a predetermined travel along which a fixedly arranged sensor unit preferably mounted on the housing (10) and a movably arranged sensor transmitter unit (27) are moving relative to each other,
the sensor unit (26) is configured to output, on the basis of the varying detection variable, a detection signal corresponding to the respective travel position,
the sensor output signal processing unit (28) is arranged to receive the detection signal output by the sensor unit (26) and to discriminate at least three travel positions on the basis of the received detection signal, wherein
the at least three travel positions comprise a closed position in which a tube portion (24) inserted in the pump is occluded, an open position in which the tube portion (24) inserted in the pump is not occluded and an intermediate position provided between the closed position and the open position, and
the sensor transmitter unit (27) is mounted on a locking bar mechanically controlled in its position by a locking lever.

2. The volumetric pump according to claim 1, wherein in the sensor system
the sensor unit (26) is disposed in the housing (10) of the pump and the sensor transmitter unit (27) is disposed on a travel position changing unit (12; 22) adjustably articulated to the housing (10), or
the sensor unit (26) is disposed on a travel position changing unit (12; 22) adjustably articulated to the housing (10) and the sensor transmitter unit (27) is disposed in the housing (10) of the pump.

3. The volumetric pump according to claim 2, wherein in the sensor system
the travel position changing unit (12; 22) is a flap unit (12) of the pump which is configured as a closing device and is arranged to occlude the tube portion (24) inserted in the pump in a closed condition against a peristaltic element disposed in the pump and conveying fluid in the tube portion (24) and to be non-occlusive against the same in an opened condition, or
the travel position changing unit (12; 22) constitutes a hinge unit (22) disposed on the flap unit (12) which adjustably articulates the flap unit (12) to the housing (10), or
the travel position changing unit is a locking bar movably coupled to the flap unit (12) of the pump, or
the travel position changing unit is a locking lever coupled to the flap unit (12) and/or to the housing (10) of the pump.

4. The volumetric pump according to claim 3, wherein in the sensor system
the detection signal of the sensor unit (26) is coupled to a rotational position of the flap unit (12) forming a first travel position and to a position of the locking lever forming a second travel position, and
the sensor output signal processing unit (28) is configured so that, only when the first travel position indicates that the flap unit (12) is completely closed and the second travel position indicates that the locking lever is provided in an intended final position corresponding to the closed position of the flap unit, an afore-detected condition of an intermediate position is cancelled.

5. The volumetric pump according to any of the preceding claims, wherein in the sensor system
the sensor unit (26) is a Hall sensor and the sensor transmitter unit (27) is a solenoid acting on the Hall sensor, or
the sensor unit (26) is a capacitive sensor and the sensor transmitter unit (27) is arranged to bring about a change of capacity corresponding to a covered travel distance of the closing device in the sensor unit (26), or
the sensor unit (26) is an inductive sensor and the sensor transmitter unit (27) is arranged to bring about a change of magnetic field corresponding to a covered travel distance of the closing device in the sensor unit (26), or
the sensor unit (26) is an optical sensor and the sensor transmitter unit (27) is arranged to bring about a change of light incidence corresponding to a covered travel distance of the closing device at the sensor unit (26), or
the sensor unit (26) is a variable-resistance element, preferably of the type of a potentiometer, and the sensor transmitter unit (27) is arranged to bring about a change of resistance corresponding to a covered travel distance of the closing device.

6. The volumetric pump according to any of the preceding claims, wherein in the sensor system
in a first travel position corresponding to a non-occluded open condition, the sensor unit (26) does not detect the presence of the sensor transmitter unit (27) and outputs a zero signal as a first detection signal to the sensor output signal processing unit (28);
in a third travel position corresponding to an occluded closed condition, the sensor unit (26) detects close presence of the sensor transmitter unit (27) by way of a strong application by the detection variable and outputs a strong sensor output signal corresponding to the strong application as a third detection signal to the sensor output signal processing unit (28); and
in a second travel position corresponding to an intermediate condition between the occluded closed condition and the non-occluded open condition, the sensor unit (26) detects a remote presence of the sensor transmitter unit (27) by way of an application by the detection variable which is weaker than the strong application, but is above the zero signal, and outputs a weaker sensor output signal corresponding to the weaker application as a second detection signal to the sensor output signal processing unit (28).

7. The volumetric pump according to claim 6, wherein in the sensor system
the sensor output signal processing unit (28) is arranged to dynamically evaluate the first, second and/or third detection signal as analogous signals along the predetermined travel.

8. The volumetric pump according to any of the preceding claims, wherein in the sensor system
the sensor output signal processing unit (28) is arranged to perform a plausibility check of the detection signal based on time behavior of the detection signal, and/or
the sensor output signal processing unit (28) is configured to evaluate the travel position by adjusting the dynamics of the detection signal to a model of the time behavior.

9. The volumetric pump according to any claims 1 to 8, wherein in the sensor system
the sensor output signal processing unit (28) is configured to output an alarm signal when the intermediate position provided between the closed position and the open position is detected to be longer than a predetermined period of time.

## Revendications

1. Pompe volumétrique avec un boîtier (10),
un volet frontal (12) qui présente un levier de fermeture à actionner manuellement, et
un système de capteur avec des capteurs sans contact pour la pompe volumétrique fonctionnant selon le principe du péristaltisme linéaire, laquelle détecte la position du volet frontal et/ou du levier de fermeture par rapport au boîtier (10) de la pompe volumétrique, dans laquelle le système de capteur présente ce qui suit :
au moins un dispositif de capteur (26) ;
au moins un dispositif d'émetteur de capteur (27) ; et
un dispositif de traitement de signal de sortie de capteur (28), dans laquelle
le dispositif d'émetteur de capteur (27) est disposé pour appliquer au dispositif de capteur (26), le long d'un trajet prédéterminé sur lequel un dispositif de capteur (26) disposé de manière fixe, de préférence au niveau du boîtier (10), et un dispositif d'émetteur de capteur (27) disposé de manière mobile se déplacent l'un par rapport à l'autre, une grandeur de détection variant en fonction d'une position de trajet ;
le dispositif de capteur (26) est configuré pour émettre un signal de détection correspondant à une position de trajet respective sur la base de la grandeur de détection variable ;
le dispositif de traitement de signal de sortie de capteur (28) est disposé et conçu pour recevoir le signal de détection émis par le dispositif de capteur (26) et pour distinguer au moins trois positions de trajet sur la base du signal de détection reçu, dans laquelle
les au moins trois positions de trajet contiennent une position fermée dans laquelle une section de tuyau (24) insérée dans la pompe est occluse, une position ouverte dans laquelle la section de tuyau (24) insérée dans la pompe n'est pas occluse, et une position intermédiaire qui se trouve entre la position fermée et la position ouverte ; et
le dispositif d'émetteur de capteur (27) est placé au niveau d'un verrou de fermeture dont la position est contrôlée mécaniquement par le levier de fermeture.

2. Pompe volumétrique selon la revendication 1, dans laquelle, dans le système de capteur
le dispositif de capteur (26) est disposé dans un boîtier (10) de la pompe et le dispositif d'émetteur de capteur (27) est disposé au niveau d'un dispositif de changement de position de trajet (12 ; 22) articulé de manière réglable au niveau du boîtier (10), ou
le dispositif de capteur (26) est disposé au niveau du dispositif de changement de position de trajet (12 ; 22) articulé de manière réglable au niveau du boîtier (10) et le dispositif d'émetteur de capteur (27) est disposé dans un boîtier (10) de la pompe.

3. Pompe volumétrique selon la revendication 2, dans laquelle, dans le système de capteur
le dispositif de changement de position de trajet (12 ; 22) est un dispositif de volet (12) de la pompe qui est configuré en tant que dispositif de fermeture et disposé pour occlure une section de tuyau (24) insérée dans la pompe dans un état fermé contre un élément péristaltique disposé dans la pompe et transportant du liquide dans la section de tuyau (24) et pour être non occlusif par rapport à celui-ci dans un état ouvert, ou
le dispositif de changement de position de trajet (12 ; 22) est un dispositif de charnière (22) au niveau du dispositif de volet (12) qui articule le dispositif de volet (12) de manière réglable au niveau du boîtier (10), ou
le dispositif de changement de position de trajet est un verrou de fermeture couplé de manière mobile au dispositif de volet (12) de la pompe, ou
le dispositif de changement de position de trajet est un levier de fermeture couplé au dispositif de volet (12) et/ou au boîtier (10) de la pompe.

4. Pompe volumétrique selon la revendication 3, dans laquelle, dans le système de capteur
le signal de détection du dispositif de capteur (26) est couplé à une position de rotation du dispositif de volet (12) formant une première position de trajet et à une position du levier de fermeture formant une deuxième position de trajet, et
le dispositif de traitement de signal de sortie de capteur (28) est configuré pour annuler un état détecté précédemment d'une position intermédiaire uniquement lorsque la première position de trajet indique que le dispositif de volet (12) est complètement fermé et que la deuxième position de trajet indique que le levier de fermeture se trouve dans une position finale prévue et correspondant à la position fermée du dispositif de volet.

5. Pompe volumétrique selon l'une quelconque des revendications précédentes, dans laquelle, dans le système de capteur
le dispositif de capteur (26) est un capteur à effet Hall et le dispositif d'émetteur de capteur (27) est un aimant sollicitant le capteur à effet Hall, ou
le dispositif de capteur (26) est un capteur capacitif et le dispositif d'émetteur de capteur (27) est disposé pour provoquer dans le dispositif de capteur (26) une modification d'une capacité correspondant à un trajet parcouru par le dispositif de fermeture, ou
le dispositif de capteur (26) est un capteur inductif et le dispositif d'émetteur de capteur (27) est disposé pour provoquer dans le dispositif de capteur (26) une modification d'un champ magnétique correspondant à un trajet parcouru par le dispositif de fermeture, ou
le dispositif de capteur (26) est un capteur optique et le dispositif d'émetteur de capteur (27) est disposé pour provoquer au niveau du dispositif de capteur (26) une modification d'une incidence de lumière correspondant à un trajet parcouru par le dispositif de fermeture, ou
le dispositif de capteur (26) est un élément à résistance modifiable, de préférence de type potentiomètre, et le dispositif d'émetteur de capteur (27) est disposé de manière à provoquer au niveau du dispositif de capteur une variation de résistance correspondant à un trajet parcouru par le dispositif de fermeture.

6. Pompe volumétrique selon l'une quelconque des revendications précédentes, dans laquelle, dans le système de capteur
le dispositif de capteur (26), dans une première position de trajet correspondant à un état ouvert non occlusif, ne détecte pas la présence du dispositif d'émetteur de capteur (27) et émet un signal nul en tant qu'un premier signal de détection au niveau du dispositif de traitement de signal de sortie de capteur (28) ;
le dispositif de capteur (26), dans une troisième position de trajet correspondant à un état fermé occlusif, détecte une présence proche du dispositif d'émetteur de capteur (27) à l'aide d'une forte sollicitation avec la grandeur de détection et émet un signal de sortie de capteur fort correspondant à la forte sollicitation en tant qu'un troisième signal de détection au niveau du dispositif de traitement de signal de sortie de capteur (28) ; et
le dispositif de capteur (26) détecte, dans une deuxième position de trajet correspondant à un état intermédiaire entre l'état fermé occlus et l'état ouvert non occlus, une présence éloignée du dispositif d'émetteur de capteur (27) à l'aide d'une sollicitation par la grandeur de détection plus faible par rapport à la sollicitation forte, mais supérieure au signal nul, et émet un signal de sortie de capteur plus faible correspondant à la sollicitation plus faible en tant que deuxième signal de détection au dispositif de traitement de signal de sortie de capteur (28).

7. Pompe volumétrique selon la revendication 6, dans laquelle, dans le système de capteur
le dispositif de traitement de signal de sortie de capteur (28) est disposé pour évaluer dynamiquement le premier, le deuxième et/ou le troisième signal de détection le long du trajet prédéterminé en tant que signaux analogiques.

8. Pompe volumétrique selon l'une quelconque des revendications précédentes, dans laquelle, dans le système de capteur
le dispositif de traitement de signal de sortie de capteur (28) est disposé pour effectuer une plausibilisation du signal de détection sur la base d'un comportement temporel du signal de détection, et/ou
le dispositif de traitement de signal de sortie de capteur (28) est configuré pour évaluer la position de trajet en comparant la dynamique du signal de détection à un modèle du comportement temporel.

9. Pompe volumétrique selon l'une quelconque des revendications 1 à 8, dans laquelle, dans le système de capteur
le dispositif de traitement de signal de sortie de capteur (28) est configuré pour émettre un signal d'alarme lorsque la position intermédiaire, qui se trouve entre la position fermée et la position ouverte, est détectée pendant une durée plus longue qu'une durée prédéterminée.
